(19) Europäisches Patentamt
European Patent Office
Office européen des brevets

(11) Veröffentlichungsnummer: **0 087 098**
**B1**

(12) # EUROPÄISCHE PATENTSCHRIFT

(45) Veröffentlichungstag der Patentschrift: **20.05.87**

(51) Int. Cl.⁴: **C 07 D 251/54**, C 07 D 251/70, A 61 K 7/44

(21) Anmeldenummer: **83101389.1**

(22) Anmeldetag: **14.02.83**

(54) **s-Triazinderivate und ihre Verwendung als Lichtschutzmittel.**

(30) Priorität: **23.02.82 DE 3206398**

(43) Veröffentlichungstag der Anmeldung: **31.08.83 Patentblatt 83/35**

(45) Bekanntmachung des Hinweises auf die Patenterteilung: **20.05.87 Patentblatt 87/21**

(84) Benannte Vertragsstaaten: **AT BE CH DE FR GB IT LI NL**

(56) Entgegenhaltungen:
**EP-A-0 046 139**
**CH-A-469 053**
**DE-A-2 121 184**
**DE-A-3 119 385**
**DE-B-1 017 616**

**Chemical Abstracts, Band 87, Nr. 12, 19 September 1977, Columbus, Ohio, USA; G.M. POGOSYAN et al. "Synthesis of 2-R-4,6-bis(p-carboxyphenylamino)-s-triazines and preparation of polymers based on them", Seite 3, Abstract Nr. 85289v**

(73) Patentinhaber: **BASF Aktiengesellschaft, Carl-Bosch- Strasse 38, D-6700 Ludwigshafen (DE)**

(72) Erfinder: **Hoppe, Udo, Dr., Lottbeker Weg 7, D-2000 Hamburg 65 (DE)**
Erfinder: **Seib, Karl, Dr., Kriemhildstrasse 36, D-6940 Weinheim (DE)**
Erfinder: **Naegele, Paul, Wiesenstrasse 9, D-6708 Neuhofen (DE)**
Erfinder: **Roland, Martin, Dr., Hasenstrasse 60, D-6750 Kaiserslautern (DE)**

**EP 0 087 098 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft die Verwendung bestimmter s-Triazine, die an den Kohlenstoffatomen durch Reste der p-Aminobenzoesaure, ihren Salzen und/oder ihren Alkyl- oder Polyethylenglykolestern substituiert sind, als Lichtschutzmittel sowohl für die menschliche Haut als auch für sonstige technische Bereiche.

Die Erfindung betrifft ferner neue s-Triazine, die an mindestens einem Kohlenstoffatom durch den Rest eines p-Aminobenzoesäureesters und an den übrigen durch die vorgenannten Reste substituiert sind.

Der als UV-B-Strahlung bezeichnete Bereich zwischen 280 und 320 nm des Sonnenlichtes oder künstlicher Lichtquellen ist bekanntlich für die Erythembildung auf der menschlichen Haut verantwortlich. Das Maximum der Wirksamkeit der UV-Strahlung für die Erythembildung liegt bei 297 nm, wenn die Strahlungsintensität für alle Wellenlängen gleich groß ist. Beim Sonnenlicht mit Strahlung unterschiedlicher Intensität ist dieses Maximum auf 308 nm verschoben.

Durch geeignete Filtersubstanzen für den UV-B-Bereich gelingt es, die Erythembildung zumindest zu verzögern. Die Pigmentbildung in der Haut, welche die Ursache für die Bräunung darstellt, soll dagegen nicht verhindert werden.

Die UV-Strahlung ist darüber hinaus auch eine wichtige Einflußgröße bei der Alterung von Polymeren und kann beispielsweise die Umwandlung bestimmter Farbstoffe oder Zersetzungen im Polymermolekül selbst bewirken, so daß auch für solche Produkte Filtersubstanzen als Stabilisatoren nahezu unentbehrlich sind.

In den vergangenen 40 bis 50 Jahren ist eine große Anzahl von Verbindungen auf ihre Filterwirkung im UV-B-Bereich untersucht worden. Bei diesen Untersuchungen speziell von Verbindungen für den Lichtschutz der menschlichen Haut mußte dabei neben der Filterwirksamkeit im Bereich der Erythembildung auch die noch zu erhaltende Durchlässigkeit im Bereich der UV-A-Strahlung, welche die Pigmentbildung bewirkt, berücksichtigt werden. Schließlich sollten solche Substanzen auch haut- und schleimhautverträglich, nicht toxisch und auch nicht durch Sauerstoff, Wärme und UV-Strahlung zersetzlich sein. In der Kosmetik wichtige Erfordernisse bestehen auch in der Lagerstabilität, Fehlen von Eigengeruch sowie in der Verträglichkeit mit den sonstigen üblicherweise verwendeten kosmetischen Rohstoffen.

In der Praxis haben sich von den zahlreichen geprüften Verbindungen im Einblick auf die vorgenannten Erfordernisse nur relativ wenige Substanzklassen bewährt - es sei in diesem Zusammenhang auf Chemische Rundschau 24, 1097 ff (1971) verwiesen. In dieser Literaturstelle werden z.B. p-Methoxyzimtsäureester, p-Aminobenzoesäureester und Hydroxybenzophenon als derartige Substanzklassen angegeben.

Ein Nachteil, der diesen und vielen an dieser Stelle nicht genannten Verbindungen anhaftet, ist ihre noch zu hohe für die optimale Wirksamkeit erforderliche Einsatzmenge, die bei kosmetischen Präparaten möglichst vermieden werden sollte, da dann auch bei Substanzen mit geringster gemessener Toxizität schädliche Nebenwirkungen nicht ausgeschlossen werden können. Das Ziel der Erfindung bestand daher darin, Substanzen aufzufinden, die bei möglichst geringer Dosierung eine möglichst große Lichtschutzwirkung entfalten und gleichzeitig auch eine gute Durchlässigkeit für die pigmentbildende UV-A-Strahlung aufweisen, und die ansonsten allen Kriterien bezüglich Stabilität, Nichttoxizität, Verträglichkeit mit den übrigen kosmetischen Hilfsstoffen und, sofern sie nicht löslich sind, auch hinsichtlich ihrer Emulgier- bzw. Dispergierbarkeit in den kosmetischen Trägerstoffen genügen.

Dieses Ziel wurde Oberraschenderweise durch die Verwendung von Verbindungen der Formel I

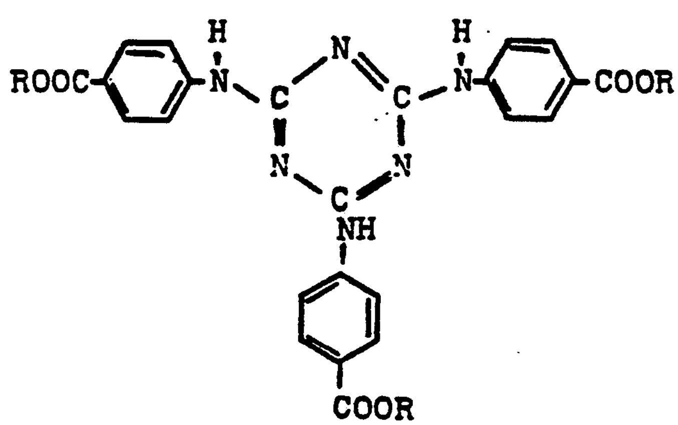

I,

in der die Reste R gleich oder verschieden sind und für Wasserstoff, K, Na, Ammonium, Mono-, Di- oder Tri-$C_1$- bis $C_4$-alkylammonium oder Mono-, Di- oder Tri-$C_2$- bis $C_4$-alkanolammonium, $C_1$- bis $C_{20}$-alkyl oder einen Polyoxyethylenrest mit 1 bis 10 Ethylenoxideinheiten, deren endständige OH-Gruppe durch einen Alkohol mit 1 bis 3 C-Atomen verethert sein kann, stehen, als Lichtschutzmittel erreicht.

Besonders gute Ergebnisse wurden mit den unter die Formel I fallenden neuen Verbindungen

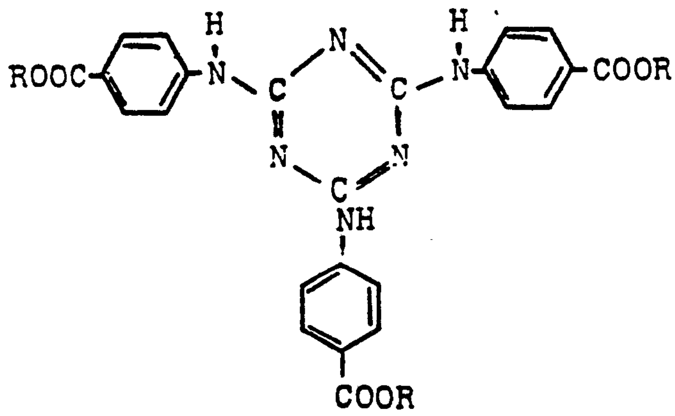

I,

in der die Reste R gleich oder verschieden und für $C_1$- bis $C_{20}$-Alkyl, einen Polyoxyethylenrest mit 1 bis 10 Ethylenoxideinheiten, deren endständige OH-Gruppe durch einen Alkohol mit 1 bis 3 C-atomen verethert sein kann, Wasserstoff, K, Na, oder Ammonium, Mono-, Di- oder Tri-$C_1$- bis $C_4$-alkylammo-nium oder Mono-, Di- oder Tri-$C_2$- bis $C_4$-alkanolammonium mit der Maßgabe stehen, daß mindestens ein Rest R für einen Alkyl- oder einen Polyoxyethylenrest steht, erreicht.

Davon sind die Verbindungen, in denen die Reste R gleich sind und für $C_6$- bis $C_{12}$-Alkyl oder einen Polyoxyethylenrest mit 1 bis 6 Ethylenoxideinheiten, deren endständige OH-Gruppe methyliert ist, stehen, besonders bevorzugt.

Es handelt sich um Umsetzungsprodukte des Cyanurchlorids oder -bromids (2,4,6-Trichlor(brom)-s-triazin) mit p-Aminobenzoesäureestern, die gegebenenfalls ganz oder teilweise zu den freien Säuren oder deren Salzen verseift oder mit Polyethylenglykolen ganz oder teilweise umgeestert sind.

Die freien Säuren sind aus Chem. Abst. Vol 67 (1977), 85 289 v bekannt - sie dienen gemäß diesem Zitat als Zwischenprodukte für weitere Synthesen, jedoch nicht als Lichtschutzmittel oder kosmetische Hilfsstoffe. Die Ester sind aber in der Literatur nicht beschrieben.

Die erfindungsgemäß zu verwendenden s-Triazinderivate können hergestellt werden aus Cyanurchlorid oder -bromid, vorzugsweise Cyamurchlorid, und einem $C_1$- bis $C_{20}$-Alkyl-ester oder einem Polyoxyethylenester mit 1 bis 10 Ethylenoxideinheiten-, deren endständige OH-Gruppe durch einen Alkohol mit 1 bis 3 C-Atomen verethert sein kann, der p-Aminobenzoesäure. Sie werden mit dem Cyanurchlorid oder -bromid im Molverhältnis 3 : 1 bis 4 : 1 umgesetzt.

Die erhaltenen Ester der Formel I, insbesondere die niederen Alkylester, können nun gewünschtenfalls umgewandelt werden, und zwar einmal durch Verseifung, wobei man die freien Säuren bzw. deren Salze erhält. Um zu neuen Verbindungen, wie sie in den Ansprüchen 2 und 3 definiert sind, zu gelangen, setzt man die Ester mit höchstens zwei Dritteln der zur Verseifung nötigen Menge an Verseifungsmitteln um. Als Verseifungsmittel wendet man. NaOH, KOH, $Na_2CO_3$, $K_2CO_3$, $NH_3$, Mono-, Di- oder Tri-$C_1$-bis $C_4$-alkylamine oder Mono-,-Di- oder Tri-$C_2$- bis $C_4$-alkanol-amine an; man erhält dann die Salze, die durch Ansäuern in die freie Säure umgewandelt werden können. Auch die direkte Umwandlung der Ester in die Säure ist mittels beispielsweise Schwefel- oder Salzsäure möglich, doch führt diese Methode häufig zur Spaltung (Verseifung) des Moleküls.

Die freie Säure mit drei Carboxylgruppen im Molekül kann im übrigen auch durch direkte Umsetzung von Cyanurchlorid oder -bromid mit p-Aminobenzoesäure erhalten werden; diese Reaktion ist aber aus dem oben genannten Chem. Abst.-Zitat bekannt.

Die freie Säure mit drei Carboxylgruppen kann, insbesondere im Falle von Polyoxyethylenresten, auch direkt verestert werden.

Des weiteren können die Ester mit niederen Alkylresten mit anderen Alkoholen nach bekannten Methoden umgeestert werden.

Bevorzugte Verbindungen im Sinne der Erfindung sind die definitionsgemäßen Ester selbst, von denen der 2-Ethylhexylester und der 3,5,5-Trimethylpentylester ("Isononylester") besonders bevorzugt sind. Von den Polyethylenglykolen sind solche mit 2 bis 6 Ethylenoxideinheiten und der Methyldiglykolester bevorzugt. Die niedrigen Ester, vor allem der Methylester, setzt man vornehmlich als Zwischenprodukt für die Herstellung der freien Säuren, Salze oder der Polyethylenglykolester ein.

Die erfindungsgemäßen Verbindungen absorbieren mit einer sehr guten Extinktion den UV-B-Bereich. Durch die Variation der Reste R können Verbindungen von bestimmter Konsistenz und mit gewünschten Eigenschaften im Hinblick auf Löslichkeit und Emulgierbarkeit hergestellt werden. Die Palette der bisher verfügbaren Lichtschutzmittel wird durch die chemisch leicht zugänglichen Verbindungen in vorteilhafter Weise bereichert. So können öl- bis wasserlösliche bzw. -emulgierbare Verbindungen hergestellt werden. Die Alkalimetall- und Ammoniumsalze sind gut wasserlöslich, wohingegen die freie Säure und vor allem die Ester mit steigender Kohlenstoffzahl öllöslich sind.

Als wasserlösliche Ammoniumsalze sind insbesondere zu nennen das Tri-(diethanolammoniumsalz) und das Tri-(triethanolammoniumsalz).

Der größte Vorteil besteht aber darin, daß die Verbindungen als UV-B-Filter mindestens doppelt so stark wirksam sind, wie die stärksten bisher bekannten Mittel, d.h. man kommt bei der Herstellung von Lichtschutzkosmetika im allgemeinen mit der halben Menge und weniger an Wirkstoff aus, um mit dem Stand der Technik vergleichbare Lichtschutzwirkungen zu erzielen.

Die Filterwirkung für den UV-B-Bereich kann allgemein in kosmetischen Formulierungen aber auch zur Stabilisierung von Kunststoffen, Farbstofformulierungen oder Lacken ausgenutzt werden. Selbstverständlich können die Verbindungen auch in Kombination mit anderen Lichtschutzmitteln zum Einsatz gelangen.

Kosmetische Präparate oder Zubereitungen enthalten die Verbindungen in allgemeinen zu 0,1 bis 10 Gew.%, vorzugsweise zu 0,2 bis 5 Gew.% - bezogen auf die Formulierungen - neben in der Kosmetik üblichen Trägerstoffen oder Verdünnungsmitteln und gegebenenfalls üblichen kosmetischen Hilfsstoffen.

Die kosmetischen Zubereitungen enthalten ansonsten die üblichen festen, halbfesten oder flüssigen Trägerstoffe oder Verdünnungsmittel, Gemische davon und gegebenenfalls sonstige übliche kosmetische Hilfsstoffe.

Von der Art des Trägers, Hilfsstoffes oder Verdünnungsmittels hängt es ab, ob das fertige lichtschutzmittelhaltige Präparat beispielsweise eine Lösung, ein Öl, eine Creme, eine Salbe, eine Lotion, ein Gel oder ein Pulver ist. Derartige Zubereitungen können beispielsweise der Zeitschrift "Fette und Seifen", 53. Jahrgang, Seiten 694 bis 699 (1951), der Zeitschrift "Seifen, Öle, Fette, Wachse", 1955, Seite 147, oder H. Janistyn, Handbuch der Kosmetika und Riechstoffe, 3. Band, (1973) entnommen werden.

Üblicherweise verwendete kosmetische Hilfstoffe, die als Zusätze in Betracht kommen, sind beispielsweise Emulgatoren, wie Fettalkoholethoxylate, Sorbitanfettsäureester oder Lanolinderivate, Verdickungsmittel, wie Carboxymethylcellulose oder vernetzte Polyacrylsäure, Konservierungsmittel und Parfüms. Grundlage für Sonnenschutzöle sind beispielsweise pflanzliche Öle, wie Erdnußöl, Olivenöl, Sesamöl, Baumwollsamenöl, Kokosöl, Traubenkernöl, Ricinusöl oder Mineralöle, wie Vaselinöl, oder insbesondere flüssiges Paraffin, synthetische Fettsäureester und Glyceride. Grundlage für Salben sind beispielsweise Vaseline, Lanolin, Eucerin oder Polyethylenglykole.

Grundlage für Cremes sind beispielsweise fettreiche Cremes, Glycerin-, Polysaccharid-, Tylosecreme, für Cremes auf Basis von Fetten und Wachsen Cetylalkohol, Lanolincreme, Kakobutter, Bienenwachs, Stearinsäure, Stearylalkohol, Glycerinmonostearat, native oder mineralische Öle und Fette.

Grundlage für Emulsionen sind beispielsweise Mischungen aus Stearylglykol, einem pflanzlichen und/oder Mineralöl, wie Mandelöl, Paraffinöl und Vaseline, und Wasser oder Mischungen aus Ethylalkohol, Wasser, Lanolin und Tragant, oder Mischungen aus Ethylalkohol, Stearin, Wasser, Tragant und Glycerin oder Mischungen aus Stearinsäure, Paraffinöl, Propyl- oder Isopropylalkohol und Wasser.

Bei der Stabilisierung von Kunststoffen, Farbstoffen und anderen lichtempfindlichen technischen Produkten, setzt man die erfindungsgemäß zu verwendenden Produkte im allgemeinen zu 0,2 bis 5 Gew.% in die entsprechenden Stoffe durch Mischen ein.

Die nun folgenden Beispiele erläutern die Erfindung, ohne sie zu beschränken.

**Beispiel 1**

16,2 Teile p-Aminobenzoesäureethylhexylester und 4,0 Teile Cyanurchlorid werden in einen Benzinschnitt mit Siedepunkten zwischen 140 und 170° C gelöst. Anschließend wird das Reaktionsgemisch 4 bis 8 Stunden unter Rückfluß zum Sieden erhitzt. Das erhaltene Reaktionsgemisch wird nach dem Erkalten aus Benzin der Siedegrenzen 120 bis 130° C umkristallisiert.

Fp 128° C, Ausbeute 70 % d.Th an 1.3.5-Trianilino-p-(carbo-2-ethylhexyl-1-oxy)-s-triazin.

UV (Ethanol):

$$E^{1\,\%}_{1\,cm}$$

= 1585 bei 313,3 nm;

$$E^{1\,\%}_{1\,cm}$$

= 456 bei 218,7 nm.

In ähnlicher Weise werden die entsprechenden n-Hexyl- und 3,5,5-Trimethylpentylester hergestellt.

Der i-Amylester wird in entsprechender Weise aus p-Aminobenzoesäure-i-amylester erhalten. Fp 177-180° C.

UV (Ethanol):

$$E^{1\,\%}_{1\,cm}$$

= 1854 bei 313,5 nm;

$$E^{1\,\%}_{1\,cm}$$

= 596 bei 217,7 nm.

Das Trikaliumsalz des 1,3,5-Tris-(p-aminobenzoesäure)-s-triazins schmilzt bei 250° C.

UV (Ethanol):

$$E^{1\,\%}_{1\,cm}$$

= 1213 bei 298,5 nm.

**Beispiel 2**

1,3,5-Trianilino-p-(carbo-ethyloxy)-s-triazin

82,6 g p-Aminobenzoesäureethylester und 30,75 g Cyanursäurechlorid werden in 1500 ml Xylol 8 Stunden unter Rückfluß am Sieden gehalten. Dabei entweicht Salzsäure. Danach wird 1 1 Xylol zugegeben und die 80-90° C heiße Suspension mit gesättigter Natriumbicarbonatlösung neutral gewaschen. Nach dem Abkühlen auf Raumtemperatur wird diese Suspension über eine Glasfilternutsche abgesaugt, die erhaltenen weißen Kristalle mit Ethanol gewaschen und getrocknet. Fp 218-223° C, Ausbeute 97 % an 1,3,5-Tris-(p-Aminobenzoesäureethylester)-triazin. UV (Ethanol):

$$E^{1\,\%}_{1\,cm}$$

= 2080 bei 313 nm;

$$E^{1\,\%}_{1\,cm}$$

= 699 bei 218 nm.

**Beispiel 3**

1,3,5-Trianilino-p-(carbo-octyl-1-oxy)-s-triazin

75 g-p-Aminobenzoesäureoctylester und 18,5 g Cyanursäurechlorid werden in 1000 ml Xylol 8 Stunden auf 140° C erhitzt. Nach Beendigung der Chlorwasserstoffentwicklung wird die Reaktionslösung auf 90° C abgekühlt und zweimal mit gesättigter Natriunhydrogencarbonatlösung sowie einmal mit Wasser gewaschen. Nach dem Abkühlen auf Haumtemperatur fallen weiße Kristalle aus, welche abgesaugt werden.

Ausbeute: 79 % d.Th., Fp: 140° C, UV (Ethanol):

$$E^{1\,\%}_{1\,cm}$$

= 1555 bei 313,3 nm

$$E^{1\,\%}_{1\,cm}$$

= 447 bei 218 nm.

**Beispiel 4**

1,3,5-Trianilino-p-(carbo-3,7-dimethyloctyl-1-oxy)-s-triazin

18,5 g Cyanursäurechlorid und 84 g p-Aminobenzoesäure-tetrahydrogeranylester werden in 1000 ml Xylol 8 Stunden auf 140°C erhitzt. Nach Beendigung der Chlorwasserstoffentwicklung wird die Reaktionslösung abgekühlt und bei 90°C zweimal mit gesättigter Natriumbicarbonatlösung sowie einmal mit Wasser gewaschen. Nach dem Abkühlen auf Raumtemperatur werden die ausgefallenen weißen Kristalle abgesaugt und getrocknet.

Ausbeute: 50 % d. Th; Fp 120°C; UV (Ethanol):.

$$E_{1\,cm}^{1\,\%}$$

= 1416 bei 313,3 nm;

$$E_{1\,cm}^{1\,\%}$$

= 418 bei 218,4 nm.

**Beispiel 5**

1,3,5-Trianilino-p-(carbo-decyl-1-oxy)-s-triazin

83 g p-Aminobenzoesäure-decylester und 18,5 g Cyanursäurechlorid werden in 1000 ml Xylol 8 Stunden auf 140°C erhitzt. Nach Beendigung der Chlorwasserstoffentwicklung wird die Reaktionslösung auf 90°C abgekühlt und zweimal mit gesättigter Natriumbicarbonatlösung sowie einmal mit Wasser gewaschen. Nach dem Abkühlen auf Raumtemperatur werden die ausgefallenen weißen Kristalle abgesaugt und getrocknet.

Ausbeute: 69 % d. Th; Fp 140°C; UV (Ethanol):

$$E_{1\,cm}^{1\,\%}$$

= 1416 bei 313,3 nm.

$$E_{1\,cm}^{1\,\%}$$

= 418 bei 217,8 nm.

**Beispiel 6**

1,3,5-Trianilino-p-(carbo-dodecyl-1-oxy)-s-triazin

18,5 g Cyanursäurechlorid und 92 g p-Aminobenzoesäuredodecylester werden in 1000 ml Xylol auf 140°C erhitzt bis kein HCl-Gas mehr entweicht. Nach 8 Stunden wird die Reaktionslösung auf 80°C abgekühlt und zweimal mit gesättigter Natriumbicarbonatlösung sowie einmal mit Wasser gewaschen. Nach dem Abkühlen auf Raumtemperatur werden die ausgefallenen Kristalle abgesaugt und getrocknet.

Ausbeute: 82 % d. Th; Fp: 139-140°C; UV (Ethanol):.

$$E_{1\,cm}^{1\,\%}$$

= 1268 bei 313,2 nm;

$$E_{1\,cm}^{1\,\%}$$

= 384 bei 218,1 nm.

**Beispiel 7**

1,3,5-Trianilino-p-(carbo-3-oxa-5-hydroxypentyl-1-oxy)-s-triazin

106 g Diethylenglykol und 123 g 1,3,5-Tris-(p-Aminobenzoe-säure)-triazin werden in 1000 ml Xylol gelöst, mit 3 g p-Toluolsulfonsäure versetzt und 10 Stunden am Rückfluß zum Sieden erhitzt. Dabei werden 18 g Wasser ausgekreist. Die erhaltene Suspension wird über eine Glasfilternutscheabgesaugt. Die erhaltenen Kristalle werden getrocknet.

Ausbeute: 89 % d. Th; Fp: >250° C; UV (DMSO):

$$E_{1\ cm}^{1\ \%}$$

= 1369 bei 313,5 nm.

**Beispiel 8**

1,3,5-Trianilino-p-(carbo-3-oxa-5-methoxy-pentyl-1-oxy)-s-triazin

75 g p-Aminobenzoesäuremethyldiglykolester und 18,5 g Cyanursäurechlorid werden in 1000 ml Xylol 8 Stunden auf 140° C erhitzt. Nach Beendigung der Chlorwasserstoffentwicklung wird auf Raumtemperatur abgekühlt und das ausgefallene Produkt abfiltriert. Anschließend wird dreimal mit Xylol gewaschen und unter vermindertem Druck getrocknet.

Ausbeute: 90 % d. Th; Fp: 129° C; UV (Ethanol):.

$$E_{1\ cm}^{1\ \%}$$

= 1616 bei 313,5 nm;

$$E_{1\ cm}^{1\ \%}$$

= 495 bei 217,7 nm.

Die nun folgenden Beispiele stellen verschiedene Anwendunigsformen für die erfindungsgemäßen Lichtschutzmittel dar.

**Beispiel 9**

Eine Lichtschutzemulsion setzt sich wie folgt zusammen

| | | |
|---|---|---|
| a) | Glycerinmonostearat | 6,0 Teile |
| | Triglyceryl einer $C_8/C_{12}$-Carbonsäure | 5,0 Teile |
| | Paraffinöl | 5,0 Teile |
| | Produkt gemäß Beispiel 1 | 1,5 Teile |
| | Talgfettalkohol + 25 Ethylenoxid (EO) | 1,0 Teile |
| | Talgfettalkohol + 6 EO | 1,0 Teile |
| | 1,2-Propylenglycol | 3,0 Teile |
| | Siliconöl | 0,2 Teile |
| | Konservierungsmittel | 0,5 Teile |
| | Parfümol | 0,2 Teile |
| | Wasser | 76,6 Teile |

b)

| | |
|---|---|
| Glycerinmonostearat | 6,0 Teile |
| Paraffinöl | 5,0 Teile |
| Isopropylmyristat | 5,0 Teile |
| Produkt gem. Beispiel 1 | 1,5 Teile |
| Talgfettalkohol + 25 EO | 2,0 Teile |
| Talgfettalkohol + 6 EO | 2,0 Teile |
| Siliconöl | 0,3 Teile |
| Konservierungsmittel | 0,5 Teile |
| Parfümöl | 0,2 Teile |
| Wasser | 74,5 Teile |

**Beispiel 10**

Öl-in-Wasser-Lichtschutzcrems weisen folgende Zusammensetzung auf:.

| | |
|---|---|
| Glycerinmonostearat | 8,0 Teile |
| Cetylalkohol | 2,0 Teile |
| Talgfettalkohol + 25 EO | 2,0 Teile |
| Talgfettalkohol + 6 EO | 2,0 Teile |
| Isopropylmyristat | 15,0 Teile |
| Produkt gem. Beispiel 1 | 1,5 Teile |
| Paraffinöl | 5,0 Teile |
| 1.2 Propylenglycol | 3,0 Teile |
| Konservierungsmittel | 0,5 Teile |
| Parfümöl | 0,2 Teile |
| Wasser | 60,8 Teile |

**Beispiel 11**

Wasser-in-Öl-Lichtschutzcrems weisen folgende Zusammensetzung auf:

| | |
|---|---|
| Isopropylmyrisat | 20,0 Teile |
| Paraffinöl | 5,0 Teile |
| mikrokristallines Wachs | 6,0 Teile |
| Sorbitansesquioleat | 4,0 Teile |
| Produkt gem. Beispiel 1 | 1,5 Teile |
| Magnesiumstearat | 1,0 Teile |
| Aluminiumstearat | 1,0 Teile |
| 1.2 Propylenglycol | 3,0 Teile |
| Konservierungsmittel | 0,5 Teile |
| Parfümöl | 0,3 Teile |
| Wasser | 57,7 Teile |

Lichtschutzschäume weisen folgende Zusammensetzung auf:

| | |
|---|---|
| Stearin | 3,0 Teile |
| Cetylalkohol | 1,0 Teile |
| Triglyceryl einer $C_8/C_{12}$-Carbonsäure | 5,0 Teile |
| Paraffinöl | 5,0 Teile |
| Produkt gem. Beisp. 1 | 1,5 Teile |
| Fettalkohol + 25 EO | 1,0 Teile |
| Siliconöl | 0,1 Teile |
| Triethanolamin | 0,3 Teile |
| 1,2 Propylenglycol | 3,0 Teile |
| Konservierungsmittel | 0,5 Teile |
| Parfümöl | 0,2 Teile |
| Wasser | 79,4 Teile |

**Patentansprüche**

1. Verwendung von Verbindungen der Formel I

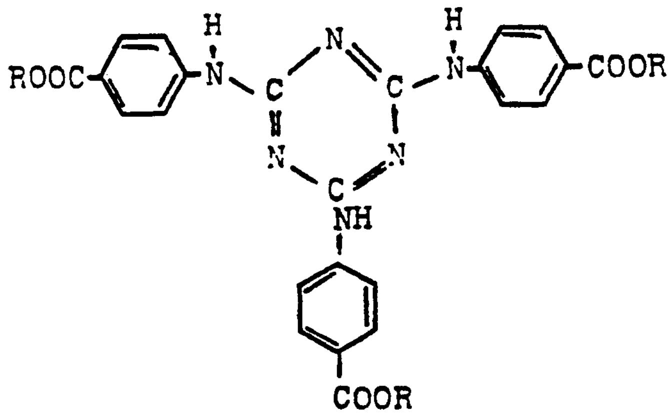

I,

in der die Reste R gleich oder verschieden sind und für Wasserstoff, K, Na, Ammonium, Nono-, Di- oder Tri-$C_1$- bis $C_4$-alkylammonium oder Mono-, Di- oder Tri-$C_2$ - bis $C_4$-alkanolammonium $C_1$- bis $C_{20}$-Alkyl oder einen Polyoxyethylenrest mit 1 bis 10 Ethylenoxideinheiten, deren endständige OH-Gruppe durch einen Alkohol mit 1 bis 3 C-Atomen verethert sein kann, stehen, als Lichtschutzmittel.

2. Verbindungen der Formel

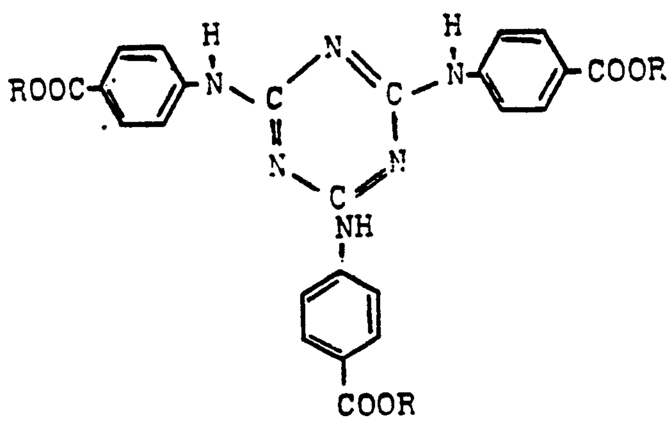

I,

in der die Reste R gleich oder verschieden und für $C_1$- bis $C_{20}$-Alkyl, einen Polyoxyethylenrest mit 1 bis 10 Ethylenoxideinheiten, deren endständige OH-Gruppe durch einen Alkohol mit 1 bis 3 C-Atomen verethert sein kann, Wasserstoff, K, Na oder Ammonium, Mono-, Di- oder Tri-$C_1$- bis $C_4$-alkylammonium oder Mono-, Di- oder Tri-$C_2$- bis $C_4$-alkanolammonium mit der Maßgabe stehen, daß mindestens ein Rest R für einen Alkyl- oder einen Polyoxyethylenrest steht.

3. Verbindungen der Formel I gemäß Anspruch 2, in der die Reste R gleich sind und für $C_6$- bis $C_{12}$-Alkyl oder einen Polyoxyethylenrest mit 1 bis 6 Ethylenoxideinheiten, deren endständige OH-Gruppe methyliert ist, stehen.

Verfahren zur Herstellung von Verbindungen der Formel I gemäß Ansprüchen 2 oder 3, dadurch gekennzeichnet, daß man Verbindungen der Formel II

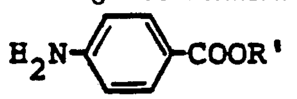

II,

in der R' für $C_1$- bis $C_{20}$-Alkyl oder einen Polyoxyethylenrest mit 1 bis 10 Ethylenoxideinheiten, deren endständige OH-Gruppe durch einen Alkohol mit 1 bis 3 C-Atomen verethert sein kann, steht, mit Cyanurchlorid oder -bromid im Molverhältnis 3 : 1 bis 4 : 1 umsetzt und gegebenenfalls den erhaltenen Ester mit höchstens zwei Drittel der zur vollständigen Verseifung erforderlichen Menge an Verseifungsmitteln in die freie Säuren bzw. deren K, Na, oder Ammonium, Mono-, Di- oder Tri-$C_1$- bis $C_4$-alkylammonium oder Mono-, Di- oder Tri-$C_2$- bis $C_4$-alkanolammoniumsalze überführt oder mit einem Alkohol R"OH, in dem R" die für R' in Formel II angegebenen Bedeutungen hat, umsetzt, oder eine freie Säure der Formel I, in der R ein Wasserstoffatom bedeutet, direkt verestert.

5. Kosmetische Zubereitung, enthaltend eine Verbindung der Formel I

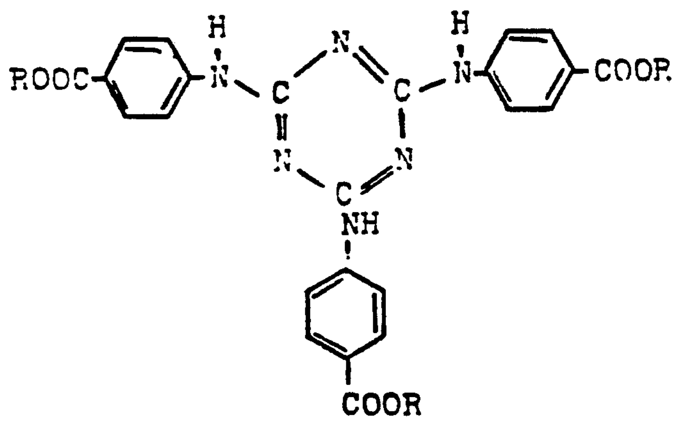

I,

in der die Reste R gleich oder verschieden sind und für Wasserstoff, K, Na, Ammonium, Mono-, Dioder Tri-$C_1$-bis $C_4$-alkylammonium oder Mono-, Di- oder Tri-$C_2$- bis $C_4$-alkanolammonium $C_1$- bis $C_{20}$-Alkyl oder einen Polyoxyethylenrest mit 1 bis 10 Ethylenoxideinheiten, deren endständige OH-Gruppe durch einen Alkohol mit 1 bis 3 C-Atomen verethert sein kann, stehen, als Lichtschutzmittel in einer Menge von 0,1 bis 10 Gew.% neben in der Kosmetik üblichen Trägerstoffen oder Verdünnungsmitteln und gegebenenfalls üblichen kosmetischen Hilfsstoffen.

**Claims**

1. The use of a compound of the formula I

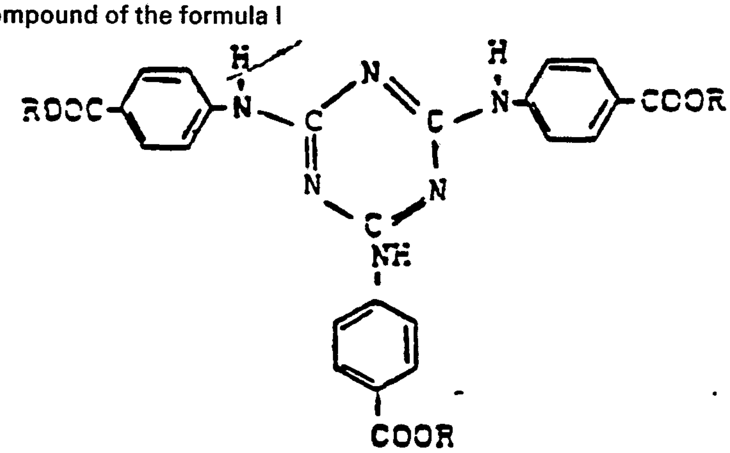

I,

where the radicals R are identical or different and are each hydrogen K, Na, ammonium, mono-, di- or tri-$C_1$-$C_4$-alkylammonium or mono-, di- or tri-$C_2$-$C_4$-alkanolammonium $C_1$-$C_{20}$-alkyl or a polyoxyethylene radical which contains from 1 to 10 ethylene oxide units and whose terminal OH group can be etherified by an alcohol of 1 to 3 carbon atoms, as a sun screen agent.

2. A compound of the formula I

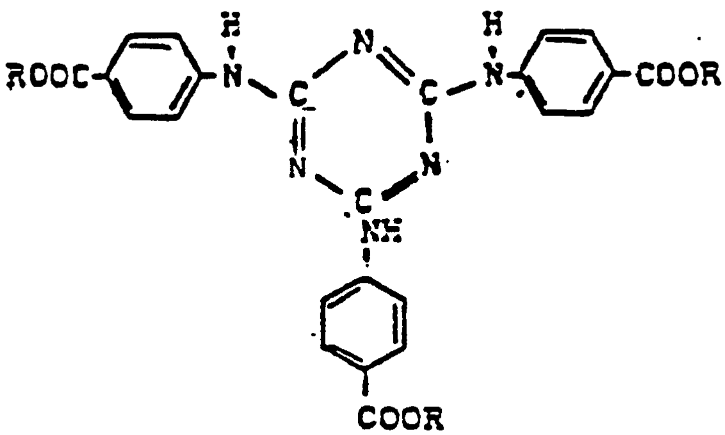

I,

where the radicals R are identical or different and are each $C_1$-$C_{20}$-alkyl, a polyoxyethylene radical which contains from 1 to 10 ethylene oxide units and whose terminal OH group can be etherified by an alcohol of 1 to 3 carbon atoms, hydrogen, K, Na, ammonium, mono-, di- or tri-$C_1$-$C_4$-alkylammonium or mono-, di- or tri-$C_2$-$C_4$-alkanolammonlum, with the proviso that at least one of the radicals R is alkyl or a polyoxyethylene radical.

3. A compound of the formula I as claimed in claim 2, where the radicals R are identical and are each $C_6$-$C_{12}$-alkyl or a polyoxyethylene radical which contains 1 to 6 ethylene oxide units and whose terminal OH group is methylated.

4. A process for the preparation of a compound of the formula I as claimed in claim 2 or 3, wherein a compound of the formula II

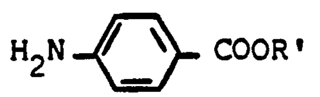

II,

where R' is $C_1$-$C_{20}$-alkyl or a polyoxyethylene radical which contains from 1 to 10 ethylene oxide units and whose terminal OH group can be etherified by an alcohol of 1 to 3 carbon atoms, is reacted with cyanuric chloride or bromide in a molar ratio of from 3 : 1 to 4 : 1, and, if desired, the ester obtained is converted, with the aid of no more than two thirds of the amount of hydrolyzing agent necessary for complete hydrolysis, into the free acid or its K, Na, ammonium, mono-, di- or tri-$C_1$-$C_4$-alkylammonium or mono-, di- or tri-$C_2$-$C_4$-alkanolammonium salts, or is reacted with an alcohol R''OH, where R'' has the meanings given for R' in formula II, or a free acid of the formula I, where R is hydrogen, is directly esterified.

5. A cosmetic formulation which contains from 0.1 to 10% by weight of a compound of the formula I

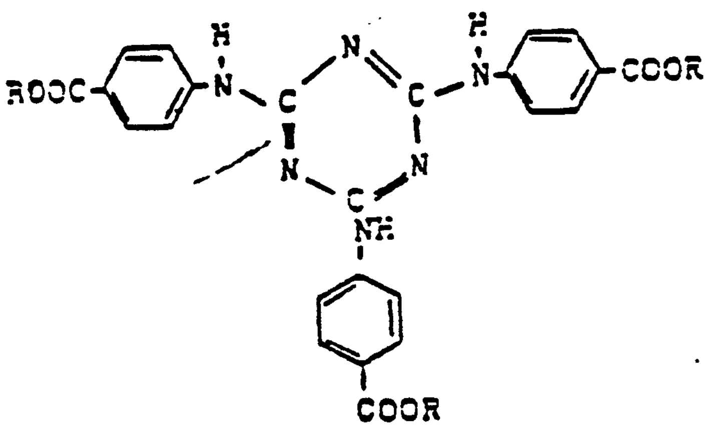

I,

where the radicals R are identical or different and are each hydrogen, K, Na, ammonium, mono-, di- or tri-$C_1$-$C_4$-alkylammonium or mono-, di- or tri-$C_2$-$C_4$-alkanolammonium, $C_1$-$C_{20}$-alkyl or a polyoxyethylene radical which contains from 1 to 10 ethylene oxide units and whose terminal OH group can be etherified by an alcohol of 1 to 3 carbon atoms, as a sun screen agent, in addition to carriers or diluents usually used in cosmetics and, if desired, conventional cosmetic auxiliaries.

**Revendications**

1. Utilisation, comme agents de protection contre la lumière, de composés de formule I

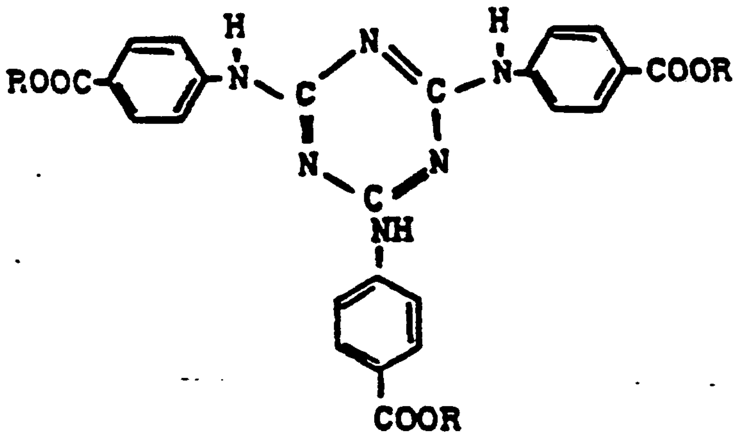

I,

dans laquelle les radicaux R sont identiques ou différents et sont mis pour des atomes d'hydrogène, de K, de Na, des groupements ammonium, mono-, di- ou tri-$C_2$- à -$C_4$-alcanolammonium, $C_1$- à $C_{20}$-alkyl ou polyoxyéthylène à 1- 10 unites oxyde d'éthylène dont le groupe OH terminal peut etre étherifié par un alcool à 1 - 3 atomes de C.

2. Composés de formule I

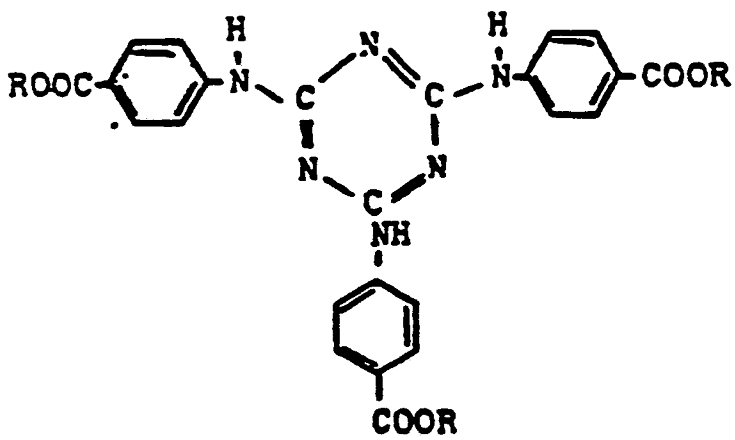

I,

dans laquelle les radicaux R sont identiques ou différents et sont mis chacun pour un radical alkyle en $C_1$ à $C_{20}$, un radical polyoxyéthylène à 1 - 10 unités oxyde d'éthylène, dont le groupe OH terminal peut être éthérifié par un alcool à 1 - 3 atomes de C, un atome d'hydrogène, de K, de Na ou um groupement ammonium, mono-, di- ou tri-$C_2$- à -$C_4$-alcanolammonium, avec cette condition qu'un radical R au moins soit mis pour un radical alkyle ou polyoxyethylène.

3. Composés de formule I selon la revendication 2, dans laquelle les radicaux R sont identiques et sont mis chacun pour un radical alkyle en $C_6$ à $C_{12}$ ou pour un radical polyoxyéthylène à 1 - 6 unités oxyde d'éthylène dont le groupe OH terminal est méthylé.

4. Procédé de préparation de composés de formule I selon la revendication 2 ou 3, caractérisé en ce qu'on fait réagir, dans un rapport molaire de 3 : 1 à 4 : 1, des composés de formule II

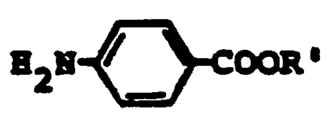

II,

dans laquelle R' est mis pour un radical alkyle en $C_1$ à $C_{20}$ ou un radical polyoxyéthylène à 1 - 10 unités oxyde d'éthylène dont le groupe OH terminal peut être éthérifié par un alcool à 1 - 3 atomes de C, avec le chlorure ou le bromure de cyanuryle et, le cas échéant, on transforme l'ester obtenu en l'acide libre ou en ses sels de K, Na ou d'ammonium, de mono-, di- ou tri-$C_1$ à $C_4$-alkylammonium ou mono-, di- ou tri-$C_2$ à $C_4$-alcanolammonium, avec une quantité d'agent saponifiant correspondant au maximum aux deux tiers de la quantité nécessaire

pour la saponification complète, ou on le fait réagir avec un alcool R''OH, R'' ayant la signification donnée pour R' dans la formule II, ou encore on estérifie directement un acide libre de formule I dans laquelle R représente un atome d'hydrogène.

5. Préparation cosmétique, contenant un composé de formule I

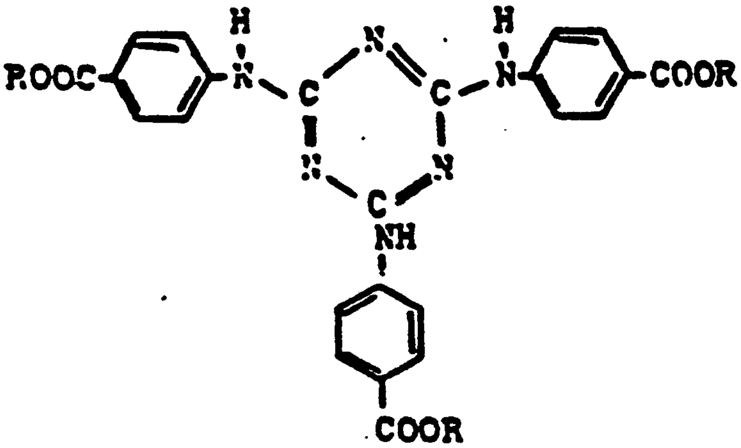

I,

dans laquelle les radicaux R sont identiques ou différents et sont mis chacun pour un atome d'hydrogène, de K, de Na, un groupement ammonium, mono-, di- ou tri-$C_2$ à $C_4$-alkylammonium ou mono-, di- ou tri-$C_2$ à $C_4$-alcanolammonium, un radical alkyle en $C_1$ à $C_{20}$ ou un radical polyoxyéthylène à 1 - 10 unités oxyde d'éthylène dont le groupe OH terminal peut être éthérifié par un alcool à 1 - 3 atomes de C, en tant qu'agent de protection contre la lumière, dans une proportion de 0,1 à 10 % en poids, outre les excipients ou diluants utilisés habituellement en cosmétique et, le cas échéant, des adjuvants cosmétiques usuels.